# FASCICULE DE BREVET EUROPEEN

(11) **EP 0 912 442 B1**
(45) Date de publication et mention de la délivrance du brevet: **23.04.2003**
(21) Numéro de dépôt: 97932858.0
(22) Date de dépôt: 08.07.1997
(51) Int. Cl.: C01B 31/24

(54) **UTILISATION DE SILICE COMME AGENT CONTROLANT LA DEGRADATION DU BICARBONATE, MELANGE RESULTANT ET SON APPLICATION**
VERWENDUNG VON KIESELSÄURE AUS DEM ABBAU VON BIKARBONAT KONTROLLIERENDEM WIRKSTOFF, MISCHUNG DARAUS UND IHRE VERWENDUNG
USE OF SILICA AS AGENT FOR CONTROLLING THE DEGRADATION OF BICARBONATE, RESULTING MIXTURE AND ITS APPLICATION

(30) Priorité: 08.07.1996 FR 9608458
(43) Date de publication de la demande: 06.05.1999
(73) Titulaire: Novacarb, 92408 Courbevoie Cédex (FR)
(72) Inventeur: GUBELMANN, Michel, F-78100 Saint-Germain-en-Laye (FR); SEGUIN, Michel, F-92200 Neuilly-sur-Seine (FR)
(74) Mandataire: Jacobson, Claude
(86) Numéro de dépôt international: FR9701234
(87) Numéro de publication internationale: WO98001390

(56) Documents cités:
- DE-A- 1 546 508
- DATABASE WPI Section Ch, Week 9231 Derwent Publications Ltd., London, GB; Class A60, AN 92-253918 XP002024881 & JP 04 170 315 A (TOSOH CORP) , 18 juin 1992

## Description

La présente invention a pour objet l'emploi de silice comme agent contrôlant la dégradation thermique du bicarbonate de métal alcalin, alcalino-terreux ou d'ammonium, ainsi que l'utilisation des mélanges silice/bicarbonate ainsi obtenus dans des applications en cosmétique et détergence.

Les bicarbonates de très fine granulométrie, c'est-à-dire inférieure ou égale à 100 µm, sont des éléments constitutifs bien connus des formulations cosmétiques et détergentes, notamment du fait de leurs propriétés abrasives, de contrôle du pH.

Cependant, ils présentent un inconvénient qui est relatif à leur température de dégradation thermique. Par dégradation thermique, on entend plus particulièrement la température à laquelle le dioxyde de carbone est dégagé. Par exemple, dans la préparation de formulations pour pâtes dentifrices, la température habituelle de mélange est de l'ordre de 50°C afin d'éviter toute dégradation du bicarbonate qu'elles contiennent. Or il serait avantageux que cette température puisse être élevée de manière à assurer une productivité améliorée du procédé de formulation des pâtes dentifrices.

En outre, le bicarbonate présente un autre inconvénient lié à son aptitude très élevée au mottage, aptitude d'autant plus marquée que la granulométrie est fine. Ceci pose notamment des problèmes lors de l'utilisation dudit bicarbonate après un stockage plus ou moins long. Il est donc nécessaire de pouvoir disposer d'un bicarbonate présentant une meilleure stabilité au stockage.

Ces problèmes et d'autres sont résolus par la présente invention qui consiste donc en l'utilisation de silice dont la taille moyenne de particules est comprise entre 8 et 30 µm en mélange avec du bicarbonate de métal alcalin, alcalino-terreux ou encore d'ammonium, dans le but de stabiliser thermiquement ledit bicarbonate, ce dernier présentant une taille moyenne de particules inférieure ou égale à 100 µm.

Il a été trouvé de façon tout à fait inattendue que le bicarbonate de très fine granulométrie pouvait être stabilisé lorsqu'il se trouvait en mélange avec de la silice, non seulement lors du stockage, mais aussi lors de son utilisation. En effet, le bicarbonate ainsi additivé présente une coulabilité améliorée par rapport au bicarbonate seul, de même qu'une capacité à motter diminuée. En outre, la gamme de température à laquelle le bicarbonate est dégradé, c'est-à-dire la gamme de température dans laquelle on constate un départ de dioxyde de carbone, est déplacée vers des températures plus élevées et la gamme est élargie.

Enfin, le mélange selon l'invention présente l'avantage de pouvoir être utilisé directement par l'utilisateur. La composition du mélange selon l'invention est en effet compatible avec des applications en cosmétique et détergence notamment, comme par exemple la formulation de pâtes dentifrices. Ceci permet à l'applicateur de le dispenser d'une étape supplémentaire de dosage de bicarbonate et de silice, tout en disposant d'un mélange ne présentant pas les inconvénients du bicarbonate seul.

Mais d'autres caractéristiques et avantages de la présente invention apparaîtront plus clairement à la lecture de la description et des exemples qui vont suivre.

L'invention a donc pour premier objectif de stabiliser thermiquement le bicarbonate de fine granulométrie.

Le bicarbonate peut être un bicarbonate de métal alcalin, alcalino-terreux, ou encore d'ammonium. Plus particulièrement, le bicarbonate stabilisé est un bicarbonate de métal alcalin ou alcalino-terreux. De préférence, le bicarbonate est un bicarbonate de sodium.

La taille moyenne des particules de bicarbonate est inférieure ou égale à 100 µm. Selon un mode de réalisation particulier de l'invention, la taille moyenne des particules de bicarbonate est comprise entre 10 et 90 µm. Plus particulièrement la taille moyenne des particules de bicarbonate est comprise entre 20 et 80 µm et de préférence entre 20 et 60 µm. La granulométrie est mesurée en utilisant un tamis AFNOR ou encore par diffraction laser à l'aide d'un granulomètre SYMPATEC.

Le bicarbonate est obtenu en général en mettant en oeuvre un procédé comprenant deux parties essentiellement. Ainsi, dans une première étape, on prépare du carbonate de métal alcalin, alcalino-terreux ou d'ammonium, selon le procédé Solvay par exemple. Dans une seconde partie, on prépare une solution aqueuse par dissolution du carbonate ainsi obtenu puis on injecte du dioxyde de carbone pour obtenir le bicarbonate.

Les bicarbonates commercialisés conviennent à la présente invention. Cependant, la présente invention est particulièrement avantageuse pour stabiliser des bicarbonates de granulométrie extra-fine.

La silice va maintenant être décrite.

Selon la présente invention, on emploie plus particulièrement une silice précipitée.

Par silice précipitée, on entend ici une silice obtenue par précipitation à partir de la réaction d'un silicate alcalin avec un acide en général inorganique à un pH adéquat du milieu de précipitation. En particulier le pH mis en oeuvre est basique, neutre ou peu acide. Le mode de préparation de la silice peut être quelconque, comme par exemple consister en l'addition d'acide sur un pied de cuve de silicate ou encore l'addition simultanée totale ou partielle d'acide ou de silicate sur un pied de cuve d'eau ou de solution de silicate, par exemple.

On peut utiliser dans la présente invention des silices de qualité abrasive ou bien épaississante. Il est à noter que la qualité de la silice est, de manière classique, dépendante des conditions dans lesquelles la précipitation est mise en oeuvre.

Selon un mode de réalisation préféré de l'invention, la silice mise en oeuvre est de qualité abrasive.

On pourrait aussi mettre en oeuvre des silices ayant subi par la suite une étape de calcination à température élevée, par exemple supérieure ou égale à 450°C.

Cependant, on préfère utiliser une silice n'ayant pas subi un tel traitement.

Selon une autre caractéristique de la silice utilisée comme stabilisant du bicarbonate, elle présente une taille moyenne des particules comprise entre 8 et 30 µm. Plus particulièrement, la taille moyenne des particules est comprise entre 8 et 25 µm et de préférence entre 10 et 20 µm. La taille moyenne des particules est déterminée, en général, par diffraction laser sur un granulomètre SYMPATEC.

De même, les silices précipitées utilisées dans la présente invention possèdent, de préférence, une surface spécifique BET comprise entre 140 et 300 m²/g, en particulier entre 210 et 250 m²/g. La surface spécifique BET est déterminée selon la méthode de BRUNAUER-EMMET-TELLER décrite dans "The Journal of the American Chemical Society", vol. 60, page 309, février 1938 et correspondant à la norme ISO 5764/1 (annexe I).

La silice employée dans la présente invention présente, de préférence, une prise d'huile DOP comprise entre 250 et 370 ml/100g, en particulier entre 300 et 350 ml/100g. La prise d'huile DOP est déterminée selon la norme ISO 787/5 en mettant en oeuvre le dioctylphtalate.

De manière avantageuse, on emploie une silice dont le volume poreux total est supérieur à 2,10 cm³/g. En général, le volume poreux total est inférieur à 5 cm³/g. Le volume poreux peut être mesuré par porosimétrie au mercure.

La silice mise en oeuvre dans la présente invention présente, présente habituellement, un pH compris entre 6,8 et 7,5. Le pH est mesuré selon la norme ISO 787/9 (pH d'une suspension à 5 % dans l'eau).

On utilise en général, comme silice précipitée, la TIXOSIL 43, la TIXOSIL 103, commercialisées par la société Rhône-Poulenc.

Selon une autre caractéristique de l'invention, la quantité de silice employée est plus précisément comprise entre 0,1 et 15 % en poids par rapport au poids de bicarbonate.

Plus particulièrement, la quantité de silice varie entre 1 et 12 % en poids. Elle est, de préférence, comprise entre 2 et 10 % en poids, et selon un mode encore plus particulier, comprise entre 4 et 10 % en poids.

La mise en contact de la silice et du bicarbonate peut être effectuée de manière tout à fait avantageuse, par un simple mélange à sec des deux produits. On utilise tout type de mélangeurs dans la mesure où ils permettent d'obtenir une répartition homogène de la silice au sein du bicarbonate. De préférence, on utilise un mélangeur à double ruban.

Ainsi, de manière tout à fait avantageuse, il n'est pas nécessaire d'utiliser des additifs supplémentaires, comme le sont notamment les agents liants, par exemple.

Il est à noter en outre que l'on ne sortirait pas du cadre de la présente invention en mélangeant les deux éléments constitutifs en présence d'un agent dispersant ceux-ci, comme les alcools par exemple, puis en séchant le mélange, dans des conditions telles que le bicarbonate ne se décompose pas. Toutefois, une telle mise en contact n'apporte pas d'avantages particuliers tout en nécessitant l'emploi d'appareillages et la mise en oeuvre d'étapes supplémentaires, qui alourdissent encore le procédé.

On a donc trouvé qu'un simple mélange mécanique de silice et de bicarbonate de métal alcalin, alcalino-terreux ou encore d'ammonium, présentait une tenue à la température améliorée.

En effet, lorsque le mélange solide selon l'invention est soumis à un traitement thermique, on constate que la dégradation du bicarbonate, mise en évidence par un départ de dioxyde de carbone, est supérieure ou égale à 95°C. En outre, on remarque que la plage de température dans laquelle le dioxyde de carbone s'échappe est élargie, ce qui va encore dans le sens d'une stabilisation thermique dudit bicarbonate.

Ceci présente un avantage intéressant lors de son utilisation dans des formulations en cosmétique et détergence, telles que les pâtes dentifrices. En effet, afin d'éviter la dégradation du bicarbonate, il est courant de formuler des mélanges comprenant du bicarbonate en suspension, à des températures proches de 50°C. En mettant en oeuvre le mélange selon l'invention, il est possible sans risque de dégagement de dioxyde de carbone, de préparer de telles formulations à des températures pouvant atteindre 70°C et plus.

On a par ailleurs remarqué que le bicarbonate ainsi additivé présentait une coulabilité améliorée avant et après stockage par rapport à du bicarbonate seul.

En effet, le mélange comprenant le bicarbonate stabilisé selon l'invention, et plus particulièrement le bicarbonate de qualité extra-fine, présente une coulabilité en instantané tel que l'indice i est d'au moins 7, et de préférence d'au moins 8,5. Un tel indice est caractéristique d'un produit coulant facilement (i compris entre 4 et 10). Il est à noter pour référence, que le même bicarbonate non additivé présente un indice i d'environ 5,5.

Par indice i, on désigne le rapport de la contrainte principale ou de la contrainte maximale pouvant être atteinte lors de l'écoulement stationnaire (σ1) à la résistance à la compression du produit (fc). Il est par ailleurs à noter que ces mesures de coulabilité sont effectuées dans une cellule de cisaillement comme la cellule de JENIKE.

Selon une variante tout à fait avantageuse de la présente invention, le mélange présentant ces indices de coulabilité en instantané comprend un bicarbonate additivé par 2 à 10 % en poids de silice par rapport au poids de bicarbonate et de préférence par 4 à 10 % en poids de silice par rapport à la même référence.

Par ailleurs, le mélange comprenant le bicarbonate traité selon l'invention, et plus particulièrement le bicarbonate de qualité extra-fine, présente une coulabilité après un stockage de 7 jours, d'au moins 3 et peut même atteindre 4,5, ce qui classe le bicarbonate additivé selon l'invention parmi les produits cohésifs, voire les produits coulant facilement. Pour référence, le même bicarbonate non additivé et après un stockage de 7 jours, présente un indice i voisin de 1, caractéristique d'un produit cohésif qui ne coule pas.

On a aussi constaté que la sensibilité de la coulabilité au stockage du mélange était diminuée par rapport au même bicarbonate non additivé.

Selon une variante tout à fait avantageuse de la présente invention, le mélange présentant ces indices de coulabilité après stockage, comprend du bicarbonate additivé par 2 à 10 % en poids de silice par rapport au poids de bicarbonate et de préférence par 4 à 10 % en poids de silice par rapport à la même référence.

En outre, comme cela a été indiqué auparavant, le bicarbonate additivé présente une aptitude au mottage considérablement diminuée par rapport au bicarbonate seul.

La présente invention a de même pour objet un mélange comprenant de la silice et du bicarbonate de métal alcalin, alcalino-terreux ou d'ammonium, ce mélange comprenant 0,1 à 15 % en poids de silice par rapport au poids de bicarbonate et ledit bicarbonate présentant une taille moyenne de particules inférieure à 100 µm.

Le bicarbonate stabilisé selon l'invention est plus particulièrement un bicarbonate de métal alcalin ou alcalino-terreux, le premier étant préféré. La présente invention est particulièrement appropriée pour stabiliser du bicarbonate de sodium.

Selon une variante de l'invention, la taille moyenne des particules de bicarbonate est comprise entre 10 et 90 µm. Plus particulièrement la taille moyenne des particules de bicarbonate est comprise entre 20 et 80 µm et de préférence entre 20 et 60 µm.

Un mode particulièrement approprié est constitué par un mélange comprenant du bicarbonate de qualité extra-fine.

Par ailleurs, selon un mode de réalisation plus particulier de l'invention, la teneur en silice varie entre 1 et 12 % en poids par rapport au poids de bicarbonate. Elle est, de préférence, comprise entre 2 et 10 % en poids, et selon un mode encore plus particulier, comprise entre 4 et 10 % en poids.

Les caractéristiques de ce mélange, telles que la coulabilité en instantané, la coulabilité après stockage à 7 jours ainsi que les propriétés de dégradation thermique du bicarbonate qu'il contient ont été explicitées auparavant et ne seront pas reprises dans cette partie.

Il est totalement surprenant que l'emploi de silice dans cette gamme bien spécifique de quantité, permette d'atteindre les objectifs de stabilisation thermique, d'augmentation de la coulabilité et de résistance au mottage, d'un bicarbonate dont la taille moyenne des particules est très fine, ce qui est un caractère aggravant de façon importante les inconvénients du bicarbonate.

Le bicarbonate de métal alcalin, alcalino-terreux ou d'ammonium selon l'invention peut être utilisé dans de nombreux domaines.

De manière avantageuse, le mélange est employé dans la préparation de formulations cosmétiques et détergentes. Selon un mode de réalisation tout particulièrement approprié de l'invention, le mélange selon invention est utilisé dans des formulations pour pâtes dentifrices.

La quantité de mélange selon l'invention peut être adaptée par l'homme du métier, en fonction des propriétés abrasives ou de composé tampon du bicarbonate présent dans ledit mélange. Elle peut donc varier dans de larges limites. Cependant, à titre illustratif, la teneur en mélange selon l'invention dans la formulation est comprise entre 10 et 50 % en poids de la formulation.

Les formulations pour pâtes dentifrices comprennent différents types d'éléments tels que des épaississants organiques ou minéraux, des composés abrasifs, des agents humectants, des tensioactifs, des additifs tels que les arômes, les adoucissants, les agents thérapeutiques, des sels, des composés tampons, ainsi que de l'eau.

En tant qu'agents épaississants organiques, on peut citer les dérivés de cellulose, tels que la carboxyméthylcellulose, l'hydroxyéthylcelluiose, l'hydroxypropylcellulose, les éthers de cellulose, les gommes comme par exemple la gomme xanthane, les alginates, les carraghénannes, les polyacrylates réticulés comme les Carbopols®.

Généralement, la teneur en agent épaississant organique est comprise entre 0,1 et 5 % en poids, de préférence entre 0,4 et 2 % en poids de la formulation.

En tant qu'agents épaississants minéraux, on peut citer les silices épaississantes, les argiles, les silicoaluminates de sodium, par exemple. Il est à noter que si le bicarbonate stabilisé selon l'invention l'est avec des silices épaississantes, la formulation pour pâte dentifrice pourra ou non comprendre une quantité supplémentaire de ce type de composé, l'adaptation étant tout à fait à la portée du formulateur.

La teneur en agent épaississant minéral est comprise entre 0 et 12 % en poids de la formulation.

Parmi les composés abrasifs convenables, on peut citer les silices abrasives, le dihydrate de calcium diphosphate, le carbonate de calcium, le bicarbonate de sodium, de calcium, le pyrophosphate de calcium, l'alumine, J'oxyde de titane, de zinc, d'étain, le talc, le kaolin. Là encore, si le bicarbonate est stabilisé avec des silices abrasives, l'homme du métier peut juger si une quantité supplémentaire en silice est ou non nécessaire pour la formulation.

La teneur en agent abrasif est habituellement comprise entre 10 et 50 % en poids de la formulation.

En ce qui concerne les tensioactifs, on peut citer sans intention de se limiter, les tensioactifs anioniques, comme notamment les sels de sodium, de magnésium, d'ammonium, d'éthanolamine des alkylsulfates en C₈-C₁₈, avec plus particulièrement le laurylsulfate de sodium, les alkyl sulfosuccinates en C₈-C₁₈, tel que par exemple le diooctyl sulfosuccinate de sodium, les alkyl sulfoacétates en C₈-C₁₈, tels que le lauryl sulfoacétate de sodium, les alkyl sarcosinates en C₈-C₁₈, comme le lauryl sarcosinate de sodium, les alkyl phosphates en C₈-C₁₈ pouvant éventuellement contenir jusqu'à 10 motifs oxyéthylénés et/ou oxypropylénés, les monoglycérides sulfatés, seuls ou en mélanges.

Comme agents tensioactifs non ioniques, on peut mentionner à titre d'exemple, les esters gras de sorbitan éventuellement polyéthoxylés, les acides gras éthoxylés, les esters de polyéthylène glycol, seuls ou en mélanges.

A titre de tensioactifs amphotères convenables, on peut citer les bétaines ou encore les sulfobétaïnes, seules ou en mélanges.

Les formulations pour pâtes dentifrices comprennent en général une quantité de tensioactifs comprise entre 0,1 et 10 %, de préférence entre 1 et 5 % en poids de la formulation.

La formulation peut comprendre en outre un ou plusieurs agents humectants comme le glycérol, le sorbitol, les polyéthylène glycols, le lactilol, le xylitol, notamment.

La teneur en agent humectant est en général comprise entre 10 et 70 % en poids de la formulation.

La formulation peut aussi comprendre des agents thérapeutiques bactéricides, anti-microbiens, anti-plaque, comme par exemple, le citrate de zinc, les polyphosphates, les guanidines.

Sont en général de même utilisés dans des formulations pour pâtes dentifrices, des agents aromatisants tels que l'essence d'anis, de menthe, de badiane, de genièvre, de cannelle, entre autres, ainsi que des édulcorants, des colorants (chlorophylle), des conservateurs, etc.

Enfin, les formulations comprennent de l'eau à raison de 5 à 50 % en poids de la formulation, de préférence de 10 à 40 % en poids de la formulation.

Ces formulations son préparées de manière conventionnelle, par mélange de chacun des éléments constitutifs de la formulation, l'avantage étant de mettre en oeuvre le bicarbonate stabilisé selon la présente invention.

Des exemples concrets mais non limitatifs de l'invention vont maintenant être présentés.

### EXEMPLE 1

### Préparation du mélange silice - bicarbonate de sodium.

On prépare un mélange comprenant 7 % en poids de silice TIXOSIL 103 (Rhône-Poulenc), par rapport au poids de bicarbonate de sodium de qualité extra-fine (Rhône-Poulenc).

L'opération est mise en oeuvre dans un mélangeur à double ruban, comprenant un ruban extérieur contre la paroi et un ruban intérieur de pas inverse.

La vitesse de rotation des deux rubans est de 31 tours / minute.

La durée du mélange est de 30 minutes.

### EXEMPLE 2

### Mesures de coulabilité en instantané.

La coulabilité en instantané a été évaluée, en utilisant une cellule de cisaillement JENIKE, pour le mélange selon l'exemple 1 après sa préparation et un bicarbonate de qualité extra-fine, démotté, échantillon témoin.

L'indice i, correspondant au rapport de la contrainte principale ou de la contrainte maximale pouvant être atteinte lors de l'écoulement stationnaire (σ1) et de la résistance à la compression du produit (fc), pour chacun des échantillons selon l'invention et témoin, ont été déterminés.

Les résultats sont rassemblés ci-dessous :

| | |
|---|---|
| témoin | 5,6 |
| mélange selon l'invention | 8,8 |

On observe une nette amélioration de l'indice de coulabilité du mélange selon l'invention.

### EXEMPLE 3

### Mesures thermogravimétriques

Les mesures des caractéristiques de dégradation thermique (départ de CO₂) du mélange de l'exemple 1 et de l'échantillon témoin, ont été effectuées en thermobalance par décomposition totale sous argon.

La gamme de température est comprise entre 0 et 500°C avec un gradient de température de + 5°C/mn.

L'appareillage est couplé à spectromètre de masse pour enregistrer la plage de température de dégagement de dioxyde de carbone.

Les gammes de températures dans lesquelles le dioxyde de carbone est dégagé sont les suivantes :

| | |
|---|---|
| témoin | 90 - 200 °C |
| mélange selon l'invention | 100 - 210 °C |

On observe un décalage significatif de la gamme de température dans laquelle le bicarbonate est décomposé, montrant que le mélange selon l'invention est stabilisé par rapport à l'échantillon témoin.

## Revendications

1. Utilisation de silice dont la taille moyenne de particules est comprise entre 8 et 30 µm, en mélange avec du bicarbonate de métal alcalin, alcalino-terreux ou encore d'ammonium, dont la taille moyenne de particules est inférieure ou égale à 100 µm, dans le but de stabiliser thermiquement ledit bicarbonate.

2. Utilisation selon la revendication précédente, **caractérisée en ce que** la taille moyenne des particules de bicarbonate est comprise entre 10 et 90 µm, de préférence entre 20 et 80 µm.

3. Utilisation selon l'une quelconque des revendications précédentes, **caractérisée en ce que** le bicarbonate présente une température de dégradation thermique supérieure à 95°C.

4. Utilisation selon l'une quelconque des revendications précédentes, **caractérisée en ce que** le mélange présente une coulabilité en instantané telle que l'indice i est d'au moins 7, et de préférence d'au moins 8,5.

5. Utilisation selon l'une quelconque des revendications précédentes, **caractérisée en ce que** le mélange présente une coulabilité après un stockage de 7 jours, d'au moins 3.

6. Utilisation selon l'une quelconque des revendications précédentes, **caractérisée en ce que** la quantité de silice est comprise entre 0,1 et 15 % en poids par rapport au poids de bicarbonate.

7. Utilisation selon la revendication 6, **caractérisée en ce que** la quantité de silice est comprise entre 1 et 12 % par rapport au poids de bicarbonate, et de préférence entre 2 et 10 % en poids par rapport à la même référence.

8. Utilisation selon l'une quelconque des revendications précédentes, **caractérisée en ce que** la surface BET de la silice est comprise entre 140 et 300 m²/g.

9. Utilisation selon l'une quelconque des revendications précédentes, **caractérisée en ce que** la silice et le bicarbonate sont mélangés à sec.

10. Mélange de silice et de bicarbonate de métal alcalin, alcalino-terreux ou d'ammonium, **caractérisé en ce qu'**il comprend 0,1 à 15 % en poids de silice par rapport au poids de bicarbonate, **en ce que** la silice présente une taille moyenne de particules comprise entre 8 et 30 µm et **en ce que** ledit bicarbonate présente une taille moyenne de particules comprise entre 20 et 80 µm.

11. Mélange selon la revendication précédente, **caractérisé en ce que** la taille moyenne des particules de bicarbonate est comprise entre 20 et 60 µm.

12. Mélange selon l'une des revendications 10 ou 11, **caractérisé en ce que** la quantité de silice est comprise entre 1 et 12 % par rapport au poids de bicarbonate, et de préférence entre 2 et 10 % en poids par rapport à la même référence

13. Mélange selon l'une des revendications 10 à 12, **caractérisé en ce que** la température de dégradation thermique du bicarbonate est supérieure à 95°C.

14. Mélange selon l'une des revendications 10 à 13, **caractérisé en ce qu'**il présente une coulabilité en instantané telle que l'indice i est d'au moins 7, et de préférence d'au moins 8,5.

15. Mélange selon l'une des revendications 10 à 14, **caractérisé en ce qu'**il présente une coulabilité après un stockage de 7 jours, d'au moins 3.

16. Application d'un mélange de silice et de bicarbonate de métal alcalin, alcalino-terreux ou encore d'ammonium selon l'une quelconque des revendications 10 à 15, dans la préparation de formulations cosmétiques et détergentes.

17. Application selon la revendication précédente, **caractérisée en ce que** l'on prépare de formulations pour pâtes dentifrices.

## Claims

1. Use of silica with an average particle size of between 8 and 30 µm, in admixture with alkali metal, alkaline earth metal or ammonium bicarbonate with an average particle size of less than or equal to 100 µm, with the objective of thermally stabilising said bicarbonate.

2. Use according to the preceding claim, **characterised in that** the average size of the bicarbonate particles is between 10 and 90 µm, preferably between 20 and 80 µm.

3. Use according to any one of the preceding claims, **characterised in that** the bicarbonate has a thermal degradation temperature above 95°C.

4. Use according to any one of the preceding claims, **characterised in that** the mixture has an instant flow behaviour such that the index i is at least 7 and preferably at least 8.5.

5. Use according to any one of the preceding claims, **characterised in that** the mixture has a flow behaviour after 7 days' storage of at least 3.

6. Use according to any one of the preceding claims, **characterised in that** the quantity of silica is between 0.1 and 15% by weight, based on the weight of bicarbonate.

7. Use according to claim 6, **characterised in that** the quantity of silica is between 1 and 12% based on the weight of bicarbonate and preferably between 2 and 10% by weight based on the same reference.

8. Use according to any one of the preceding claims, **characterised in that** the BET surface of the silica is between 140 and 300 m²/g.

9. Use according to any one of the preceding claims, **characterised in that** the silica and bicarbonate are mixed dry.

10. Mixture of silica and alkali metal, alkaline earth metal or ammonium bicarbonate, **characterised in that** it comprises 0.1 to 15% by weight of silica based on the weight of bicarbonate, **in that** the silica has an average particle size of between 8 and 30 µm and **in that** the bicarbonate has an average particle size of between 20 and 80 µm.

11. Mixture according to the preceding claim, **characterised in that** the average size of the bicarbonate particles is between 20 and 60 µm.

12. Mixture according to one of claims 10 and 11, **characterised in that** the quantity of silica is between 1 and 12% based on the weight of bicarbonate, preferably between 2 and 10% by weight based on the same reference.

13. Mixture according to one of claims 10 to 12, **characterised in that** the thermal degradation temperature of the bicarbonate is above 95°C.

14. Mixture according to one of claims 10 to 13, **characterised in that** it has an instant flow behaviour such that the index i is at least 7 and preferably at least 8.5.

15. Mixture according to one of claims 10 to 14, **characterised in that** it has a flow behaviour after 7 days storage of at least 3.

16. Use of a mixture of silica and alkali metal, alkaline earth metal or ammonium bicarbonate according to any one of claims 10 to 15 in the preparation of cosmetic and detergent formulations.

17. Use according to the preceding claim, **characterised in that** formulations for toothpastes are prepared.

## Patentansprüche

1. Verwendung von Kieselerde, deren mittlere Teilchengröße zwischen 8 und 30 Mikrometer liegt, in Mischung mit Alkalimetall-, Erdalkalimetall- oder Ammonium-Bicarbonat, dessen mittlere Teilchengröße 100 Mikrometer oder weniger beträgt, zum Zweck einer thermischen Stabilisierung des Bicarbonats.

2. Verwendung nach dem vorhergehenden Anspruch, **dadurch gekennzeichnet, dass** die mittlere Größe der Bicarbonatteilchen zwischen 10 und 90 Mikrometer, vorzugsweise zwischen 20 und 80 Mikrometer, liegt.

3. Verwendung nach einem der vorhergehenden Ansprüche, **dadurch gekennzeichnet, dass** das Bicarbonat eine über 95°C liegende Temperatur des thermischen Abbaus aufweist.

4. Verwendung nach einem der vorhergehenden Ansprüche. **dadurch gekennzeichnet, dass** das Gemisch eine solche Sofortvergießbarkeit aufweist, dass der Index i wenigstens 7 und vorzugsweise wenigstens 8,5 beträgt.

5. Verwendung nach einem der vorhergehenden Ansprüche, **dadurch gekennzeichnet, dass** das Gemisch nach siebentägiger Lagerung eine Vergießbarkeit von wenigstens 3 aufweist.

6. Verwendung nach einem der vorhergehenden Ansprüche, **dadurch gekennzeichnet, dass** bezogen auf das Bicarbonatgewicht die Menge an Kieselerde zwischen 0,1 und 15 Gewichtsprozent beträgt.

7. Verwendung nach Anspruch 6, **dadurch gekennzeichnet, dass** bezogen auf das Bicarbonatgewicht die Menge an Kieselerde zwischen 1 und 12 Gewichtsprozent beträgt und bezogen auf die gleiche Bezugsgröße vorzugsweise zwischen 2 und 10 Gewichtsprozent beträgt.

8. Verwendung nach einem der vorhergehenden Ansprüche, **dadurch gekennzeichnet, dass** die BET-Oberflache der Kieselerde zwischen 140 und 300 Quadratmeter/Gramm beträgt.

9. Verwendung nach einem der vorhergehenden Ansprüche, **dadurch gekennzeichnet, dass** die Kieselerde und das Bicarbonat trocken gemischt werden.

10. Gemisch von Kieselerde und Alkalimetall-, Erdalkalimetall- oder Ammonium-Bicarbonat, **dadurch gekennzeichnet, dass** es bezogen auf das Bicarbonatgewicht 0,1 bis 15 Gewichtsprozent Kieselerde umfasst, die Kieselerde eine zwischen 8 und 30 Mikrometer liegende mittlere Teilchengröße und das Bicarbonat eine zwischen 20 und 80 Mikrometer liegende mittlere Teilchengröße aufweist.

11. Gemisch nach dem vorhergehenden Anspruch, **dadurch gekennzeichnet, dass** die mittlere Größe der Bicarbonatteilchen zwischen 20 und 60 Mikrometer liegt.

12. Gemisch nach einem der Ansprüche 10 oder 11, **dadurch gekennzeichnet, dass** bezogen auf das Bicarbonatgewicht die Menge an Kieselerde zwischen 1 und 12 % und bezogen auf die gleiche Bezugsgröße vorzugsweise zwischen 2 und 10 Gewichtsprozent beträgt.

13. Gemisch nach einem der Ansprüche 10 bis 12, **dadurch gekennzeichnet, dass** die Temperatur des thermischen Abbaus des Bicarbonats über 95°C liegt.

14. Gemisch nach einem der Ansprüche 10 bis 13, **dadurch gekennzeichnet, dass** seine Sofortvergießbarkeit einen solchen Wert aufweist, dass der Index i wenigstens 7, vorzugsweise wenigstens 8,5 beträgt.

15. Gemisch nach einem der Ansprüche 10 bis 14, **dadurch gekennzeichnet, dass** seine Vergießbarkeit nach siebentägiger Lagerung wenigstens 3 beträgt.

16. Anwendung eines Gemisches von Kieselerde und Alkalimetall-, Erdalkalimetall- oder Ammonium-Bicarbonat nach einem der Ansprüche 10 bis 15 bei der Herstellung von kosmetischen Stoffen oder Detergentien.

17. Anwendung nach dem vorhergehenden Anspruch, **dadurch gekennzeichnet, dass** Stoffe für Zahncremes hergestellt werden.
